# EUROPÄISCHE PATENTANMELDUNG

(11) **EP 0 773 041 A2**
(43) Veröffentlichungstag der Anmeldung: **14.05.1997**
(21) Anmeldenummer: 96114426.8
(22) Anmeldetag: 10.09.1996
(51) Int. Cl.: A61N 5/06

(54) **Gerät zur fotodynamischen Bestrahlung**

(30) Priorität: 08.11.1995 DE 29517716 U
(71) Anmelder: Herbert Waldmann GmbH & Co., 78056 Villingen-Schwenningen (DE)
(72) Erfinder: Waldmann, Gerhard, 78083 Dauchingen (DE)
(74) Vertreter: Patentanwälte Westphal, Buchner, Mussgnug Neunert, Göhring

(57) **Zusammenfassung**

Gerät zur fotodynamischen Bestrahlung mit einer in einem Reflektor (18) sitzenden Lampe (16) und einer im Strahlenweg angeordneten Filtereinheit (40). Das Gerät weist eine Dosiereinrichtung zur genauen Dosierung der vom Gerät an einen Patienten abgegebenen Strahlungsenergie auf, die vorzugsweise eine zeituhr- oder rechnergesteuerte, den Strahlenweg versperrende Abblendeinrichtung (46) aufweist.

## Beschreibung

Die Erfindung betrifft ein Gerät zur fotodynamischen Bestrahlung. Die fotodynamische Bestrahlung eines Patienten wird zu therapeutischen Zwecken angewendet. Es sind zahlreiche Geräte dieser Art bekannt, mit denen es jedoch schwierig ist, die auf den Patienten angewendete Strahlungsenergie genau zu dosieren.

Die Aufgabe der Erfindung besteht daher in der Schaffung eines Geräts der eingangs genannten Art, das eine genaue Dosierung der Strahlungsenergie bei einfacher Handhabung und Wartung zuläßt.

Diese Aufgabe wird erfindungsgemäß durch die Merkmale des Anspruchs 1 gelöst.

In den Unteransprüchen sind vorteilhafte Ausgestaltungen der Erfindung unter Schutz gestellt. So kann gemäß Anspruch 2 die Dosiereinrichtung aus einer Abblendeinrichtung im Strahlenweg bestehen, die durch Zeituhr oder Rechner gesteuert wird. Die Ansprüche 3 und 4 beziehen sich auf besondere Ausgestaltungen der Abblendeinrichtung. Es ist aber auch möglich, durch die Zeituhr oder den Rechner die Brenndauer der Lampe zu steuern und dadurch eine Dosierung zu ermöglichen. Eine Abblendeinrichtung gemäß den Ansprüchen 2 bis 4 ist jedoch deshalb vorzuziehen, weil dann die Lampe nicht häufig ein- und ausgeschaltet werden muß und weiterbrennen kann, während die Abblendeinrichtung geschlossen ist.

Nach Anspruch 5 ist es aber auch möglich, unmittelbar am Behandlungsort, d.h. am Patienten, ein Strahlungsdosimeter anzubringen, das über ein Kabel mit dem Bestrahlungsgerät verbunden ist. Wenn das Strahlungsdosimeter eine vorbestimmte ausreichende Strahlungsenergie gemessen hat, kann es die Dosiereinrichtung und damit die Abschaltung oder Abblendung der Lampe steuern.

Vielfach ist es zur Einstellung der für die Dosierung wichtigen Beleuchtungsstärke am Behandlungsort wichtig, einen bestimmten Abstand zwischen der Austrittsöffnung des Geräts und dem Behandlungsort einzustellen. Nach Anspruch 6 kann hierzu ein Abstandsmesser verwendet werden, wobei Anspruch 7 sich auf eine bestimmte Ausführungsform dieses Abstandsmessers bezieht.

Die Ansprüche 8 bis 16 betreffen schließlich weitere zweckmäßige Ausgestaltungen des Geräts, die eine bequeme Handhabung und Wartung und somit eine sichere und reproduzierbare Dosierung der Bestrahlungsenergie ermöglichen.

Anhand der Figuren wird eine Ausführungsform der Erfindung näher erläutert. Es zeigt:
- Fig.1: einen schematischen Längsschnitt durch das Gerät,
- Fig.2: eine teilweise in Längsrichtung geschnittene Seitenansicht der im Gerät gemäß Fig.1 verwendeten Lampe mit Fokussiereinrichtung,
- Fig.3: eine schematische Seitenansicht der im Gerät gemäß Fig.1 verwendeten Abblendeinrichtung,
- Fig.4: eine schematische Seitenansicht einer im Gerät gemäß Fig.1 verwendeten Filtereiheit,
- Fig.5: eine vergrößerte Teilaufsicht auf den in Figur 4 mit V bezeichneten Teilbereich der Fig.4 in Längsrichtung des Geräts und
- Fig.6: einen Teilschnitt senkrecht zur Längsrichtung des Gehäuses des Geräts.

Innerhalb eines langgestreckten Gehäuses 10, dessen Wände sich parallel zu einer Längsachse 12 erstrecken, ist mittels einer Fassung 14 eine Lampe 16 geeigneter Bauart gehaltert. Die Lampe 16 wird von einem parabolischen Reflektor 18 umfaßt, der das Licht der Lampe 16 parallel zur Längsachse 12 abstrahlt. Bei einer zweckmäßigen Ausführungsform kann der Reflektor 18 einen Durchmesser bzw. eine Breite von etwa 28 cm und parallel zur Längsachse 12 eine Länge von etwa 26 cm haben.

Das rückwärtige bzw. obere Ende des Gehäuses ist durch eine mit Luftaustrittsöffnungen 20 versehene Kappe 22 abgeschlossen. Auch in das vordere bzw. untere Ende des Gehäuses 10 ist eine Abschlußkappe 24 eingesetzt, die eine mittige Lichtaustrittsöffnung 26 enthält. Zum Schutz der inneren Teile des Geräts ist die Lichtaustrittsöffnung 26 an ihrer Innenseite mit einer abnehmbaren, lichtdurchlässigen Schutzscheibe 28 verschlossen.

Auf einer Seite des Gehäuses 10 ist ein größtenteils aus Lochblech bestehender Kasten 30 angebracht, der zwei übereinander angeordnete, motorisch angetriebene Gebläse 32 und 34 enthält. Die durch das Lochblech des Kastens 30 gemäß den eingezeichneten Pfeilen angesaugte Luft wird von den Gebläsen durch eine obere Öffnung 36 in den Bereich des Reflektors 18 und der Lampe 16 und durch eine untere Öffnung 38 in den Bereich einer nachfolgend zu schildernden, allgemein mit 40 bezeichneten Filtereinheit geblasen.

Die Filtereinheit 40 ist als herausnehmbare Einschubeinheit mit einem mehrlagigen Gestell 42 ausgebildet, in das die für die jeweilige Behandlung erforderlichen Filterscheiben 44 parallel zueinander eingesetzt werden können.

Zwischen Filtereinheit 40 und Lichtaustrittsöffnung 26 ist eine allgemein mit 46 bezeichnete Abblendeinrichtung in eine quer im Gehäuse angeordnete Tragwand 48 eingesetzt. Die Abblendeinrichtung 46 wird ebenfalls nachfolgend näher erläutert.

Durch eine Öffnung 50 in der vorderen Abschlußkappe 24 des Gehäuses 10 ist ein Ende 52 eines in der Abschlußkappe 24 drehbar gelagerten Rollmaßstabes 54 zur Abstandsmessung zwischen Lichtaustrittsöffnung 26 und Behandlungsort herausziehbar und kann im Sinn des Doppelpfeils 56 verschoben werden. Selbstverständlich läßt sich bei einem etwas kostspieligeren Gerät die Abstandmessung auch mittels einer elektronischen Abstandssensoreinrichtung durchführen.

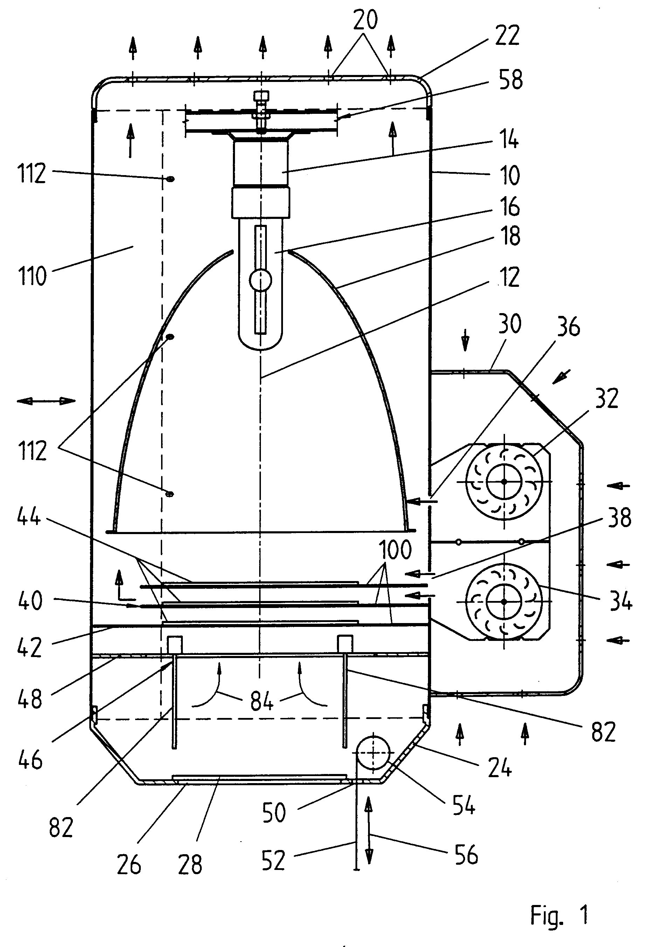

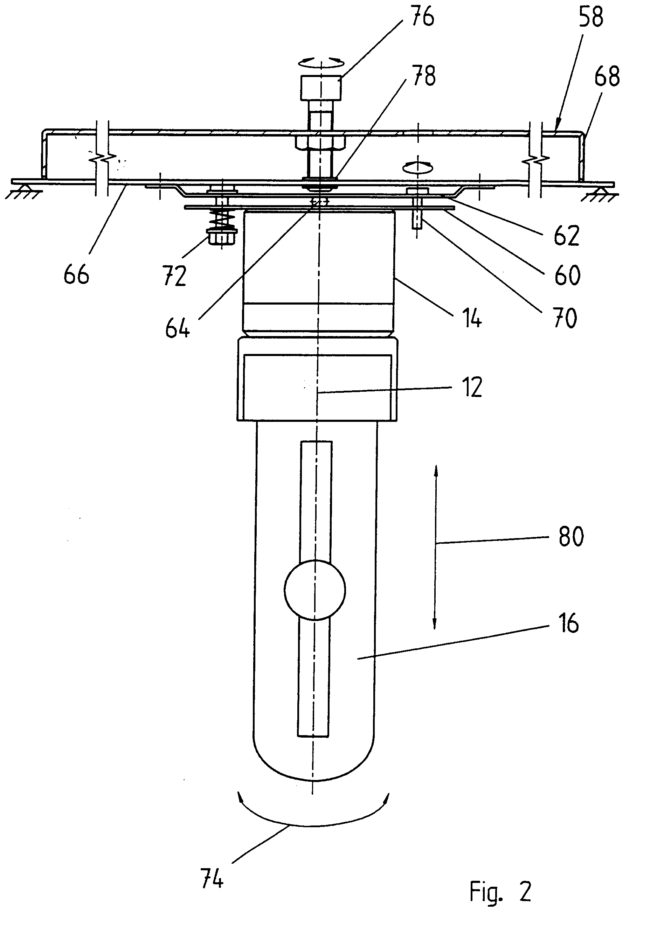

Im rückwärtigen oder oberen Abschnitt des Gehäuses 10 ist die Fassung 14 der Lampe 16 auf einer allgemein mit 58 bezeichneten Fokussiereinrichtung gelagert, die nachfolgend anhand der Figuren 1 und 2 näher erläutert wird.

Zunächst ist festzustellen, daß die Fokussiereinrichtung 58, die Lampe 16, der Reflektor 18 und die Lage der Luftaustrittsöffnungen 20 so aufeinander abgestimmt sind, daß möglichst kein direktes Licht von der Lampe 16 durch die Luftaustrittsöffnungen 20 nach außen fällt. Die Fokussiereinrichtung 58 weist ein vorderes Zwischenblech 60, auf welchem die Fassung 14 der Lampe 16 befestigt ist, sowie ein dazu etwa paralleles rückwärtiges Trägerblech 62 auf. Das Trägerblech 62 ist an einer nur an ihrem äußeren Rand gehäusefest eingespannten Membranplatte 66 befestigt, die ihrerseits mit einer topfähnlich ausgebildeten Halteplatte 68 an ihrer Rückseite oder Oberseite verbunden ist.

Zwischen dem Zwischenblech 60 und dem Trägerblech 62 ist im Bereich der Längsachse 12 eine Kugel 64 eingefügt. Eine Seite des Zwischenblechs 60 ist mittels einer in der Trägerplatte 62 angebrachten Stellschraube 70 einstellbar, während die gegenüberliegende Seite mittels einer Federdruckschraube 72 gehalten ist. Statt der einen dargestellten Stellschraube 72 können auch mehrere (nicht gezeigte) Stellschrauben verwendet werden. Durch Betätigung der Stellschraube 72 erfolgt eine Verschwenkung der Lampe 16 gemäß dem Doppelpfeil 74 um eine senkrecht zur Längsachse 12 stehende Achse. In der Halteplatte 68 ist ebenfalls eine Stellschraube 76 in Längsrichtung festgelegt, deren verstellbares Ende 78 an der Membranplatte 66 befestigt ist. Durch Betätigung der Stellschraube 76 wird daher die Mitte der Membranplatte 66 und somit die daran befestigte Trägerplatte 62 in Längsrichtung verschoben, wodurch die Lampe 16 in Richtung des Doppelpfeils 80 verstellt wird. Durch diese beiden Verstellmöglichkeiten wird eine äußerst genaue Fokussierung der Lampe im gehäusefesten Reflektor 18 erreicht.

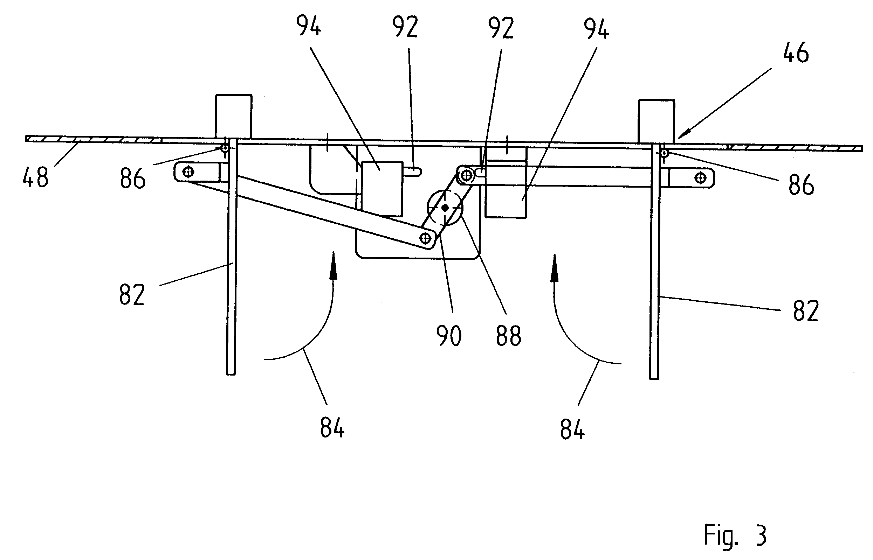

In Figur 3 ist die in Fig.1 gezeigte Abblendeinrichtung 46 vergrößert dargestellt. Die Abblendeinrichtung 46 weist zwei in der geöffneten Stellung gemäß Fig.3 parallel zur Längsachse 12 angeordnete lichtundurchlässige Klappen 82 auf, die zum Absperren des Strahlenweges des Geräts gemäß den Pfeilen 84 in eine nicht dargestellte, senkrecht zur Längsachse 12 angeordnete Stellung um ihre Drehlager 86 eingeschwenkt werden können. Ein durch einen Antriebsmotor 88 angetriebenes Kurbelgetriebe 90 bewirkt die Verschwenkung der Klappen 82, wobei die beiden Endstellungen mittels durch Druckstifte 92 betätigter Endschalter 94 eingestellt werden. Der Antriebsmotor 88 kann mit einer geeigneten Elektronik des Geräts verbunden sein, um die Verschwenkung der Klappen 82 mit genauer Zeitsteuerung durchführen zu können.

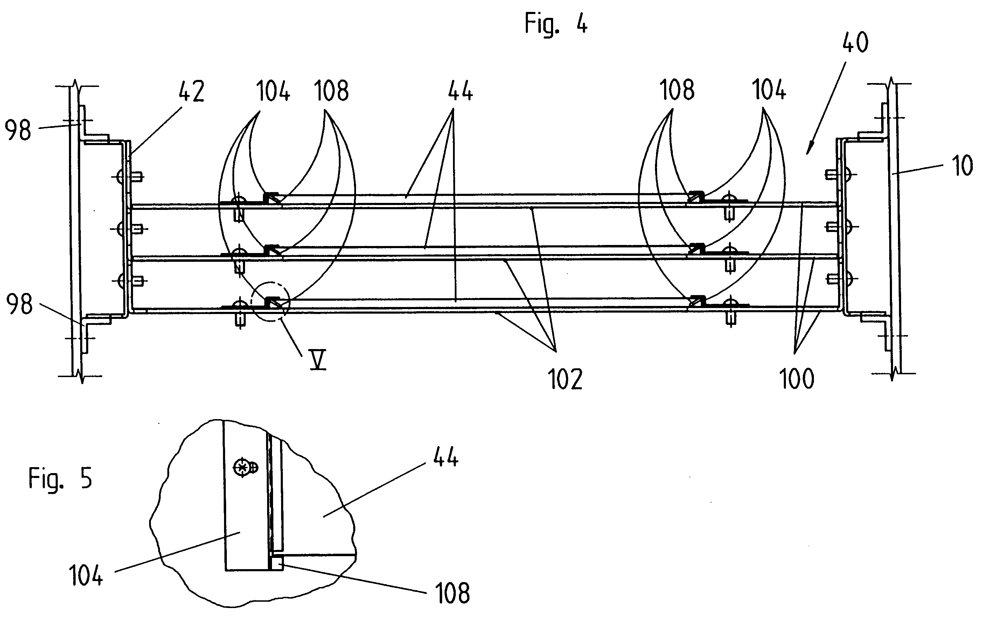

In den Figuren 4 und 5 ist die in dem Gerät gemäß Fig.1 verwendete Filtereinheit 40 ausführlicher dargestellt. Diese ist als einheitlicher Filtereinschub mit einem Gestell 42 ausgebildet, das durch geeignete Halter 98 am Gehäuse 10 festgelegt werden kann. Das Gestell 42 weist mehrere parallele Filterrahmen 100 auf, deren jeder eine Mittelöffnung 102 enthält. Ferner sind an diametral gegenüberliegenden Stellen jedes Filterrahmens 100 etwa z-förmige Profilstücke 104 befestigt, in welche jeweils eine Filterscheibe 44 eingeschoben werden kann. In Einschubrichtung der Filterscheiben 44 können die Filterscheiben nach dem Einschieben durch am Filterrahmen 100 oder an den z-förmigen Profilstücken 104 befestigte Haltenasen 108 festgelegt sein.

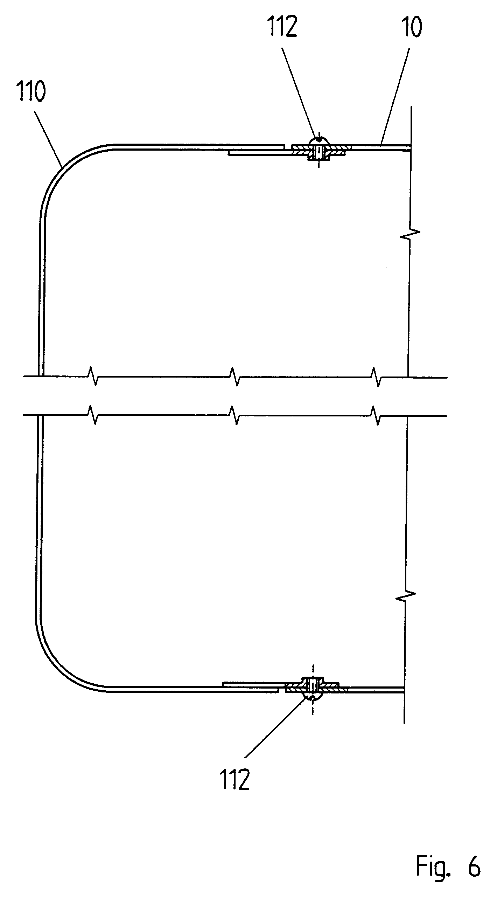

Damit eine schnelle und einfache Wartung und ggfs. Auswechslung sämtlicher Baugruppen des Geräts im Innern des Gehäuses 10 in einfacher Weise möglich ist, ist gemäß Fig.6 ein sich über die ganze Länge des Gehäuses erstreckender Teil 110 der Gehäusewand mittels leicht lösbarer Schrauben 112 oder anderer lösbarer Befestigungsmittel am übrigen Gehäuse 10 befestigt. Durch Abnahme des Gehäusesteils 110 läßt sich in einfacher Weise der Innenraum des Gehäuses 10 zu dem genannten Zweck freilegen und ebenso einfach wieder schließen.

In nicht näher dargestellter Weise kann der gesamte Reflektor 18 in Längsrichtung in etwa 40 bis 50 Facetten unterteilt sein, die sich dicht aneinander anschließend vom rückwärtigen oder oberen Ende bis zum unteren oder vorderen Ende des Reflektors erstrecken. Die Facetten können ihrerseits wieder in Querrichtung in leicht konkav gewölbte Einzelfacetten unterteilt sein. Die Gesamtwölbung bleibt dabei parabolförmig, so daß sich eine sehr gleichmäßige Beleuchtungsstärke des Behandlungsortes ergibt.

## Patentansprüche

1. Gerät zur fotodynamischen Bestrahlung, mit einem Gehäuse (10), einer im Gehäuse angeordneten Lampe (16), einem die Lampe umgebendenden Reflektor (18), einer im Strahlenweg von Lampe (16) und Reflektor (18) angeordneten Filtereinheit (40) und einer nach der Filtereinheit (40) angeordneten Lichtaustrittsöffnung (26) des Gehäuses (10), gekennzeichnet durch eine Dosiereinrichtung zur genauen Dosierung der vom Gerät an einen Patienten abgegebenen Strahlungsenergie.

2. Gerät nach Anspruch 1, dadurch gekennzeichnet, daß die Dosiereinrichtung eine zeituhr- oder rechnergesteuerte im Strahlenweg innerhalb der Lichtaustrittssöffnung (26) angeordnete, den Strahlenweg versperrende Anblendeinrichtung (46) aufweist.

3. Gerät nach Anspruch 2, dadurch gekennzeichnet, daß die Abblendeinrichtung aus einer in den Strahlenweg einfahrbaren Jalousie besteht.

4. Gerät nach Anspruch 2, dadurch gekennzeichnet, daß die Abblendeinrichtung (46) in den Strahlenweg einklappbare Klappen (82) aufweist, die über ein Kurbelgetriebe (90) mittels eines Elektromotors (88) antreibbar sind.

5. Gerät nach einem der vorangehenden Ansprüche, dadurch gekennzeichnet, daß es ein mittels Kabel angeschlossenes, am Behandlungsort anzubringendes Strahlungsdosimeter aufweist, das mittels der effektiv gemessenen Strahlungsenergie am Behandlungsort die Dosiereinrichtung steuert.

6. Gerät nach einem der vorangehenden Ansprüche, dadurch gekennzeichnet, daß es zur Einstellung eines vorbestimmten Bestrahlungsabstandes einen mechanischen oder elektronischen Abstandsmesser aufweist.

7. Gerät nach Anspruch 6, dadurch gekennzeichnet, daß der Abstandsmesser aus einem ausziehbaren Rollmaßstab (52) besteht.

8. Gerät nach einem der vorangehenden Ansprüche, dadurch gekennzeichnet, daß die Lichtaustrittsöffnung (26) durch eine Schutzscheibe (28) verschlossen ist.

9. Gerät nach einem der vorangehenden Ansprüche, dadurch gekennzeichnet, daß die Kappe des Gehäuses (10) Luftaustrittsöffnungen (20) aufweist, die so angeordnet sind, daß der Lichtaustritt durch die Luftaustrittsöffnungen (20) verschwindend klein ist.

10. Gerät nach einem der vorangehenden Ansprüche, dadurch gekennzeichnet, daß ein Seitenwandteil (110) des Gehäuses (10) vom übrigen Gehäuse lösbar ist, durch dessen Entfernung sämtliche zu wartenden Baugruppen innerhalb des Gehäuses (10) zugänglich sind.

11. Gerät nach einem der vorangehenden Ansprüche, dadurch gekennzeichnet, daß der Reflektor (18) einen Außendurchmesser von etwa 28 cm und in Längsrichtung eine Länge von etwa 26 cm besitzt.

12. Gerät nach einem der vorangehenden Ansprüche, dadurch gekennzeichnet, daß der Reflektor (18) in Längsrichtung in etwa 40 bis 50 Facetten unterteilt ist, die ihrerseits in Querrichtung in leicht konkav gewölbte Einzelfacetten unterteilt sind.

13. Gerät nach einem der vorangehenden Ansprüche, dadurch gekennzeichnet, daß die Lampe (16) bezüglich des gehäusefesten Reflektors (18) in Richtung der Längsachse (12) und senkrecht zu dieser fokussierbar ist.

14. Gerät nach Anspruch 13, dadurch gekennzeichnet, daß zur Fokussierung eine mittels Stellschrauben (70,76) einstellbare Fokussiereinrichtung (58) vorgesehen ist.

15. Gerät nach einem der vorangehenden Ansprüche, dadurch gekennzeichnet, daß zur getrennten Zwangsbelüftung des Reflektor- und Lampenbereichs (16,18) und der Filtereinheit (40) je ein durch getrennte Gehäuseöffnungen (36, 38) einblasendes Gebläse (32,34) vorgesehen ist, wobei die beiden Gebläse (32,34) in einem an die Außenseite des Gehäuses (10) angesetzten Lochblechkasten (30) angeordnet sind.

16. Gerät nach einem der vorangehenden Ansprüche, dadurch gekennzeichnet, daß die Filtereinheit (40) ein aus dem Gehäuse (10) herausnehmbares Einschubgestell (42) aufweist, in das wahlweise unterschiedliche Filterplatten (44) einsetzbar sind.
